# EUROPEAN PATENT APPLICATION

(11) **EP 2 149 380 A1**
(43) Date of publication of application: **03.02.2010**
(21) Application number: 08161384.6
(22) Date of filing: 29.07.2008
(51) Int. Cl.: A61K 38/08, A61K 38/10, A61K 38/17, A61P 25/28

(54) **Veterinary immunotherapy compositions for the Aged Related Cognitive Dysfunction.**

(71) Applicant: Medivet Pharma, S.L., 08510 Masies de Roda Barcelona (ES)
(72) Inventor: Closa Boixeda, Josep Maria, 08006, Barcelona (ES); Font Utset, Artur, 08023, Barcelona (ES); Llobera Mahy, Juan, 08014, Barcelona (ES); Llobera Mahy, Pau, 07004, Palma de Mallorca (Baleares) (ES); Mahy Gehenne, Josette-Nicole, 08328, Alella (Barcelona) (ES); Mascort Boixeda, Juan Maria, 08192, Sant Quirze del Vallès (Barcelona (ES); Pugliese, Marco, 08810, Sant Pere de Ribes (Barcelona) (ES); Puigdollers Masallera, Josep, 08510, Masies de Roda (Barcelona) (ES); Riba Casellas, Jordi, 08500, Vic (Barcelona) (ES); Rodríguez Alluè, Manuel, 08980, Sant Feliu de Llobregat (Barcelon (ES); Vivet Dordal, Gemma, 08510, Masies de Roda (Barcelona) (ES)
(74) Representative: ABG Patentes, S.L.

(57) **Abstract**

Peptide sequences as agents for curing, preventing and/or diagnosing in a non-human mammal a disease associated with diffuse Abeta plaques depositions in the CNS, said disease selected from the group consisting in Involutive Depression, Confusional Syndrome, Dysthymia and Cognitive Dysfunction Syndrome (CDS). Veterinary vaccine compositions with said peptides are also provided, as well as antibodies raised to the peptide sequences.

## Description

The present invention relates to peptide sequences and antibodies raised against these peptide sequences as agents for curing cognitive dysfunction-related diseases in non-human mammals. This invention regards to the field of veterinary diseases, especially for attending companion, domestic and livestock animals.

### BACKGROUND ART

With the presence of improved standard of veterinary care, veterinarians are seeing and treating more geriatric animals in their clinical practice. In most countries, the number of companion animals is rapidly increasing. For example, data from 1997 calculate in approximately 52.5 million the number of pet dogs and 57 millions of cats in the United States; and in 7.3 million (14%) of dogs and 6.27 million (11%) of cats the 11 year-old or older. Presently, these numbers are some 20% higher. The same occurs in Europe, where live some 38 million of dogs and 36.6 million of cats and a 25-30% of these animals are considered old.

Companion, domestic and livestock animal ageing represent a complex biological process, characterized by a progressive modification of tissues and cells with a gradual loss of adaptive capacity, and it is associated with progressive and irreversible changes in the body systems. Presently, the pathogenesis of normal and altered ageing is viewed as a multi-factored event in which oxidative stress plays a pivotal role. Although these changes are usually considered individually, the elderly animal is seldom afflicted with a single disease but rather with varying degrees of organ dysfunction. Pet owner complaints include cognitive changes that describe geriatric behavioral changes not solely due to a general medical condition such as infection, organ failure or neoplasm. These behavioral changes fall mostly into four main categories: a) loss of cognition and recognition, b) loss of housetraining, c) disorientation and d) changes in the sleep-wake cycle.

A large number of studies demonstrated that cognitive ability in non-human mammals decreases with age following specie-specific patterns, especially in dogs. Based on the predominance of affective or cognitive dysfunctions, a diagnosis of Dysthimia, Cognitive Dysfunction Syndrome (CDS), Involutive depression or Confusional Syndrome is done.

The typical clinical sign of dysthymia in animals is the loss of the ability to evaluate the relationship between a length of a passage and your own body. The dysthymic animal tends to force the way and may well remain trapped during hours moaning. Any attempt to foreign aid may trigger aggressive responses. Other clinical signs of dysthymia are going to wrong side of doors; appearing to forget previously learned tasks getting stuck in corners and behind furniture.

The term cognitive dysfunction syndrome (CDS) means the age-associated decline in the cognitive abilities of an animal that cannot be attributed to an unrelated general medical condition such as neoplasia, infection, or organ failure. Symptoms of cognitive dysfunction syndrome include, but are not limited to, altered interaction with family members, changes in sleep-wake cycle, decreased activity level, memory loss and inappropriate elimination. The Involutive depression includes depression, lethargy, and symptoms of cognitive dysfunction syndrome. The Confusional syndrome includes daze, hesitation, wandering aimlessly and symptoms of cognitive dysfunction syndrome.

In all these clinical presentations, reports of retraction of cerebral gyri and widening of sulci together with an increase in ventricular volume are described. Microscopically, lipofuscin storage, polyglucosan body, specific neuronal loss and β-amyloid (Abeta) plaque deposition are also common. In addition, these changes frequently occur in aging animals.

The cause of CDS is unknown. Studies have shown that its symptoms increase with age, and many pathological changes occur in aging dogs and cats that can theoretically lead to CDS. One such change, which has been correlated with CDS in dogs, is the formation of β-amyloid plaques in the Central Nervous System (CNS). Another change is the decline in activity of several neurotransmitters, including serotonin, acetylcholine, norepinephrine, and dopamine. Still other potential causes of CDS include, but are not limited to, elevated monoamine oxidase B (MAO-B) activity located in astroglia, and oxidation of central nervous system lipid membrane.

Nowadays the diagnosis of CDS is performed based on an indirect evaluation of the dog behavior through a formal questionnaire to the owner (Pugliese et al., "Severe cognitive impairment correlates with higher cerebrospinal fluid levels of lactate and pyruvate in a canine model of senile dementia " (2005), Prog. Neuropsychopharmacol. Biol. Psychiatr-2005, 29:603-610). This new cognitive test allows, in a reduced time, the discrimination between light and severe cognitive impaired behavior in companion dogs, ruling out the need of a specific strain to be housed during years and trained in controlled conditions. Our test, filled out by a veterinary neurologist, consisted of 16 items. For each item, score 1 indicates the normality of the specific behavior, and score 2, 3, 4 and 5 the degree of abnormal behavior. The final total score reflects the cognitive status of the animal: a 16-79 score range was allows the classification of the animals into three groups: 1) young control animals (YC); 2) dogs with light cognitive deficits (LCD); 3) dogs with severe cognitive deficits (SCD). In addition, cerebrospinal fluid (CSF) lactate, pyruvate and potassium concentrations relate with the degree of cognitive deficit. This reflects a specific relationship between availability of energy substrates and canine age-related cognitive impairment in which astroglia plays a key role and relates with MAO-B altered activity.

As in dogs, felines present a variety of behavioral changes with aging that are consistent with specific cognitive dysfunction still to be fully characterized. These signs include: inappropriate urination, including spraying and house soiling, aggression towards people and intraspecies aggression, overactivity, excessive vocalization mainly during the night, altered-sleep wake cycle, fear or anxiety, ingestive behavior, scratching furniture. In elder cats two cognitive diseases related to aging may be diagnosed, an Involutive Depression and a Dysthymia of the aging cat.

A major part of research in animal cognitive function and neuropathology is largely dedicated to elucidation of the molecular events responsible of their cognitive impairment and neuronal loss. A wide variety of age-related changes have been described in the nervous system of many non-human species. The most consistently observed brain lesions in canine, feline and polar bear mostly reflects diffuse deposits of Abeta (β-amyloid) peptide and their inability to form paired helical filaments (PHF), develop neurofibrillary tangles and mature neuritic plaques and to activate microglia. Together with the age-related cognitive decline, these brain lesions represent the major factors implicated in neurodegeneration. Together with canine aging, Abeta deposition in its early phase participates of the specific neuronal loss and then matures as a diffuse plaque, -never as a dense-core Abeta plaque- in absence of any infiltrating hyperactive microglia. In these animals, a correlation between the degree of cognitive deficit, four stages (stages I-IV) of the diffuse plaque maturation, and expression of two astroglial markers, S100beta and GFAP has been established.

The Abeta protein, a 4 kDa peptide, 42 to 43 amino acids in length, is produced by abnormal cleavage of a transmembrane protein, the amyloid precursor protein (APP) of unknown function, and is highly toxic to neurons. Several studies indicated that APP plays an essential role in the onset of the disease. The presence of β-amyloid (Abeta) plaque caused by anomalous processing of the APP protein constitutes a central event in the pathogenesis of the non-human mammal cognitive deficit, such as dogs and cats. The protein is sequentially cleaved by means of the action of alpha, beta and gamma secretases. In this pathological process, APP is anomalously processed, resulting in insoluble peptides, with 40-42 amino acids (Abeta 1-40 and Abeta 1-42), which tend to aggregate as fibrils and are deposited as diffuse plaques. In these animals, the diffuse plaques present specific characteristics in the way that they are the end product of the pathological process, they do not adopt a beta-pleated-sheet conformation, for what they are Congo red and thioflavine negative, and contain more Abeta1-42 than Abeta1-40. These plaques also contain epitopes within Abeta1-17, Abeta17-24, and Abeta1-28.

Plaque maturation is arrested at the diffuse stage because a specific ongoing damaging process develops in these animals without any microglia activation and no extensive astrogliosis. The expression of some neurotrophic factor also presents non-human mammal specificities (Pugliese et al., "Canine cognitive deficit correlates with diffuse plaque maturation and S100beta (-) astrocytosis but not with insulin cerebrospinal fluid level. " Acta Neuropathol - 2006, 111:519-528).

Results have shown the formation of diffuse β-amyloid plaques throughout all cortical grey matter layers of canine brain. This process begins when animals are 8 year-old dogs, and increases with age and cognitive deficit severity, following a four-stage maturation process of the diffuse plaques. A positive correlation between plaque density, stage of Abeta deposition and cognitive deficit has also been demonstrated. Plaque formation and maturation were not associated with modifications in synaptic protein expression. Indeed, the active mitogen activated protein kinase (MAPK/ERK-P), p38 kinase (p38-P) expression, and tau hyper-phosphorylation in neighboring cell processes remains unchanged. These results argue for regarding Abeta diffuse plaque formation and tau hyperphosphorylation as independent events, in opposite as what occurs in human aging and Alzheimer's disease (Pugliese et al., "Diffuse beta-amyloid plaques and hyperphosphorylated tau are unrelated processes in aged dogs with behavioral deficits", Acta Neuropathologica - 2006, 112:175-183.).

Unlike in the rodent, the amino acid sequence of Abeta of polar bear, rabbit, cow, cat, sheep, pig, guinea pig, dolphins and Iberian lynx deduced from amplified cDNA is identical to the canine and monkey sequence and these species accumulate β-amyloid plaques with age. In addition to these characteristics these aged non-human mammals exhibit high levels of brain oxidative stress.

Different therapeutic approaches have been selected for cognitive dysfunction research therapeutics in the veterinary field: neuroprotective, restorative or anti-amyloid approaches.

Examples of neuroprotective and restorative approaches are the treatments with selegiline and nicergoline. Selegiline inhibits the monoamine oxidase B (MAO-B), thus reducing oxidative damage and enhancing the activity of dopamine. Besides, nicergoline produces brain vasodilatation, increasing neuronal activity and decreasing neuronal degeneration. All these chronic therapies present severe side effects, such as vomiting, diarrhea or changes in behavior, such as hyperactivity and restlessness.

The amyloid hypothesis (approach) posits that Abeta peptides derived from the proteolytic processing of the transmembrane APP, initiate the process leading to neuronal dysfunction and clinical impairment. Within the anti-amyloid therapeutic approaches, one is based on the identification of small molecules that could inhibit one or another step of amyloid induced cascade in now well under way. Of particular interest are other approaches that attempt to interfere with the aggregation of Abeta 1-42 peptides by decreasing their secretion from neuronal and glial cells or by inhibiting the toxicity that these extracellular aggregates produce on neurons and glial cells and their processes.

The transport of Abeta between CNS and plasma plays a role in the regulation of brain amyloid levels, with Abeta being rapidly transported from cerebrospinal fluid (CSF) to plasma. Therefore of particular interest is the anti-amyloid approach that relies in active vaccination with Abeta peptides that can alter the dynamic equilibrium between the plasma, CSF and ultimately the CNS.

Current evidences suggest that increased plasma Abeta levels following active or passive immunization is due to an active peripheral clearance named as sink effect. The induction or direct plasma addition of anti-Abeta or other antipeptide antibodies serve to quickly and efficiently increase the clearance of CNS Abeta. Generation of this peripheral sink mechanism via induction or administration of antibodies is then useful for treating abnormal Abeta protein accumulation in the CNS, resulting in a net efflux of central Abeta to the periphery for its further rapid elimination.

Regarding the treatment by means of vaccination, some studies have been conducted in non-human primates and dogs. In the document "A two-year study with fibrillar beta-amyloid (Abeta) immunization in aged canines: effects on cognitive function and brain Abeta", J Neuroscience-2008;28(14):3555-66, Head et al. immunized aged beagles (8.4-12.4 years) with fibrillar Abeta (1-42) formulated with aluminum salt (Alum) for 2.4 years. Cognitive testing during this time revealed no improvement in measures of learning, spatial attention, or spatial memory. After extended treatment (22 vaccinations), maintenance of prefrontal-dependent reversal learning ability was observed. In the brain, levels of soluble and insoluble Abeta 1-40 and Abeta 1-42 and the extent of diffuse plaque accumulation was significantly decreased in several cortical regions. Although Head et al. indicate that by immunizing animals before extensive Abeta deposition and cognitive decline to prevent oligomeric or fibrillar Abeta formation may have a greater impact on cognition and also more directly evaluate the role of Abeta on cognition in canines, the study resulted in neither beneficial effect on cognition, nor any functional outcome benefit. No beneficial effect on cognition was observed since the dogs were not diagnosed as suffering from Cognitive Dysfunction Syndrome.

Moreover, vaccination with the complete peptide Abeta 1-40 or Abeta 1-42 may favor severe allergic reactions on the subjects, and prompt the immediate interruption of the treatment. Therefore, there is a need to finf some vaccines that while being immunogenic, do not present undesirable side effects.

Besides their veterinarian interest, no anti-amyloid treatment is currently available for non-human mammals. It is highly desirable to find successful therapies to improve the quality of live of aging pet and livestock animals suffering from a disease such as in Involutive Depression, Confusional Syndrome, Dysthymia and Cognitive Dysfunction Syndrome (CDS). As described above, up to date several attempts have been made to avoid or dissociate Abeta deposition in the dog's brain, thus hoping to obtain an improvement of canine cognitive behavior. Nevertheless, a previous attempt has not been successful. In contrast with the findings mentioned above, the present invention provides a solution to the mentioned problems proposing new sequences derived from the Abeta protein to be used in a veterinary vaccine composition for the treatment of disease in which diffuse Abeta plaques depositions are observed in the CNS.

### SUMMARY OF THE INVENTION

It has been surprisingly found that some sequences derived from canine Abeta protein induce immunity without carrying the drawbacks mentioned before, for instance producing severe anaphylactic shocks or otherwise allergic reactions in a non-human mammal. Moreover, these sequences promote beneficial effects on cognition in the non-human mammals suffering from a disease in which diffuse Abeta plaques depositions is observed in the brain.

The non-human mammal is selected from the group consisting of dogs, cats, monkeys, bears, rabbits, cows, sheep, pigs, guinea pigs, dolphins and Iberian lynxes. In all these non-human mammals the peptide sequence of the Abeta 1-40 and Abeta 1-42 has a high homology degree, comprised between 80% and 100%.

The canine Abeta 1-40 peptide is considered as less immunogenic than the Abeta 1-42 and, as a result, it has not been seriously considered for use in active vaccination of pet and livestock animals. However, the inventors surprisingly found that selected sequences derived from canine Abeta 1-40 peptide, induce a relevant immunogenic response in the treated subjects while presenting the advantage of not inducing an allergic reaction, at the same time they promote beneficial effects on cognition disorders.

Therefore, a first object of the present invention is a peptide which comprises SEQ ID NO 1 or fragments thereof as agent for curing, preventing and/or diagnosing in a non-human mammal a disease associated with diffuse Abeta plaques depositions in the brain, said disease selected from the group consisting of Dysthymia, Involutive Depression, Confusional Syndrome and Cognitive Dysfunction Syndrome (CDS).

The term "which comprises SEQ ID NO 1" means that the peptide includes SEQ ID NO 1 and some variations in number and type of amino acids of said sequence, for example two or three amino acids more, added at any of its N-and /or C-terminal ends, or the substitution of at least an amino acid for another, with the proviso that all these variations lead to conservative modifications that do not change the global structure of the peptide and/or its function.

The peptide SEQ ID NO 1 or fragments thereof can be used as immunogenic agents for the treatment and or prevention of the diseases associated with diffuse Abeta plaques depositions in the brain. As immunogenic agent or antigen, it must be understood any substance that prompts the generation of antibodies and can cause an immune response.

Diffuse Abeta plaques are protein aggregates of the Abeta peptide presenting the characteristics that they do not adopt a beta-pleated-sheet conformation. The configuration adopted in a diffuse plaque is a low-density aggregate of fibrils.

A second object of the invention is a veterinary composition comprising effective therapeutic amounts of the SEQ ID NO 1 or fragments thereof, as those defined by SEQ ID NO 2, SEQ ID NO 3, SEQ ID NO 4, SEQ ID NO 5, SEQ ID NO 6, SEQ ID NO 7, SEQ ID NO 8 and SEQ ID NO 9, or mixtures of these fragments, and any acceptable carrier, excipient and/or adjuvant.

A third object of the invention is an antibody or fragments thereof raised by the SEQ ID NO 1 or fragments of this sequence, said antibodies specifically recognizing SEQ ID NO 1, as agents for curing, preventing and/or diagnosing in a non-human mammal a disease associated with diffuse Abeta plaques depositions in the brain, said disease selected from the group consisting of Involutive Depression, Confusional Syndrome, Dysthymia and Cognitive Dysfunction Syndrome (CDS)

By "antibody" is meant a whole antibody, including without limitation a chimeric, caninized, canine, recombinant, transgenic, grafted and single chain antibody, and the like, or any fusion protein, conjugates, fragments, or derivates thereof that contain one or more domains that selectively bind canine Abeta peptide of SEQ ID NO 1 or Abeta peptide of other species having a degree of homology with the canine comprised between 80% and 100%. Antibody thereby includes a whole immunoglobulin molecule, a monoclonal antibody, a chimaeric antibody, a caninized antibody, a canine antibody, or an immunologically effective fragment of any of these. An antibody fragment means an Fv, a disulfide linked Fv, scFv, Fab, Fab', or F(ab')2 fragment, which are well known in the art. The expression "anti-Abeta antibody" means an antibody that recognizes or binds canine Abeta peptide.

A fragment of an antibody means any part of the same with adequate size and conformation to bind to an epitope of the peptide comprising SEQ ID NO 1.

Another object of the invention is a method of obtaining an antibody that specifically interacts with SEQ ID NO 1, which comprises the steps of:
a) administering in a non-human mammal a peptide which comprises SEQ ID NO 1 or fragments thereof; and
b) obtaining the antibody from the serum of said non-human mammal.

Another aspect of the invention is a veterinary composition comprising effective therapeutic amounts of antibodies and/or fragments thereof raised by the SEQ ID NO 1, or fragments of this sequence, and any acceptable carrier, excipient and/or adjuvant.

The invention relates also to the use of peptide comprising SEQ ID NO 1 or fragments thereof for the preparation of a medicament for the treatment of a disease associated with diffuse Abeta plaques depositions in the brain, said disease selected from the group consisting of Involutive Depression, Confusional Syndrome, Dysthymia and Cognitive Dysfunction Syndrome (CDS).

The invention also relates to a method of treatment of a disease associated with diffuse Abeta plaques depositions in the brain, said disease selected from the group consisting in Involutive Depression, Confusional Syndrome, Dysthymia and Cognitive Dysfunction Syndrome (CDS), in which a therapeutic amount of SEQ ID NO 1 or its fragments is administered to a non-human mammal in need thereof.

Another aspect of the invention is the use of antibodies or fragments thereof, raised by the SEQ ID NO 1 or its fragments, for the preparation of a medicament for the treatment of a disease associated with diffuse Abeta plaques depositions in the brain, said disease selected from the group consisting of Involutive Depression, Confusional Syndrome, Dysthymia and Cognitive Dysfunction Syndrome (CDS).

The invention also relates to a method of treatment of a non-human mammal wherein therapeutic amounts of antibodies or fragments thereof, raised by the SEQ ID NO 1 or fragments of this peptide are administered in a non-human mammal suffering from a disease, said disease associated with diffuse Abeta plaques depositions in the brain, and selected from the group consisting of Involutive Depression, Confusional Syndrome, Dysthymia and Cognitive Dysfunction Syndrome (CDS).

Another object of the invention is the use of the antibodies for the preparation of an agent for the diagnosis or prognosis of a disease associated with diffuse Abeta plaques depositions in the brain, said disease selected from the group consisting of Involutive Depression, Confusional Syndrome, Dysthymia and Cognitive Dysfunction Syndrome (CDS).

These and other objects of the present invention will be further described in the detailed description section that follows, and they are not intended to be limiting of the present invention. Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention.

Furthermore, the present invention covers all possible combinations of particular and preferred groups described hereinabove.

### BRIEF DESCRIPTION OF THE DRAWINGS

With the aim of aiding to the comprehension of the present invention, the following figures are enclosed, showing the results achieved with the peptides and antibodies of the invention.

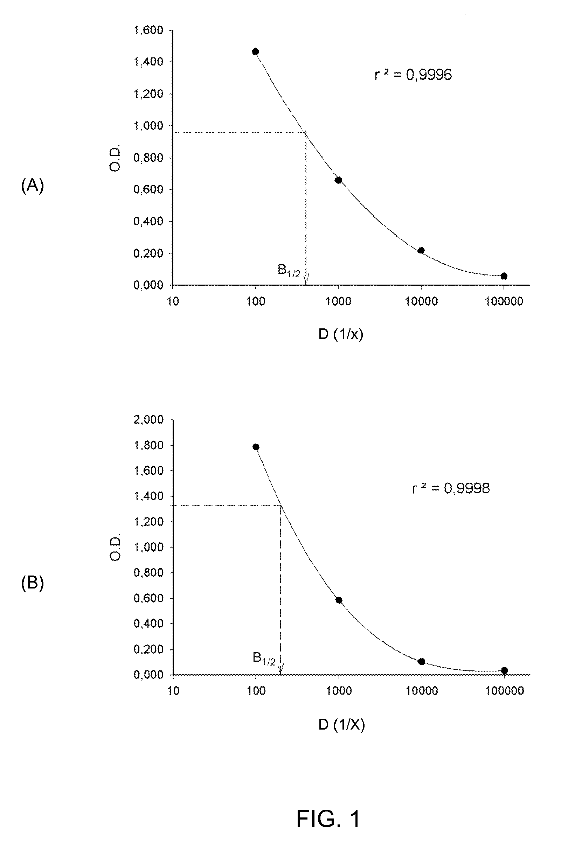

FIG. 1 is a set of graphics with the anti-Abeta IgG titration of serum samples of rabbits immunized with SEQ ID NO 3 (A) and SEQ ID NO 1 (B). In X-axis the inverse (1/X) of the serum dilution (D) is indicated. OD means Optical Density. B_{1/2} is the serum dilution that presents half of the maximal binding.

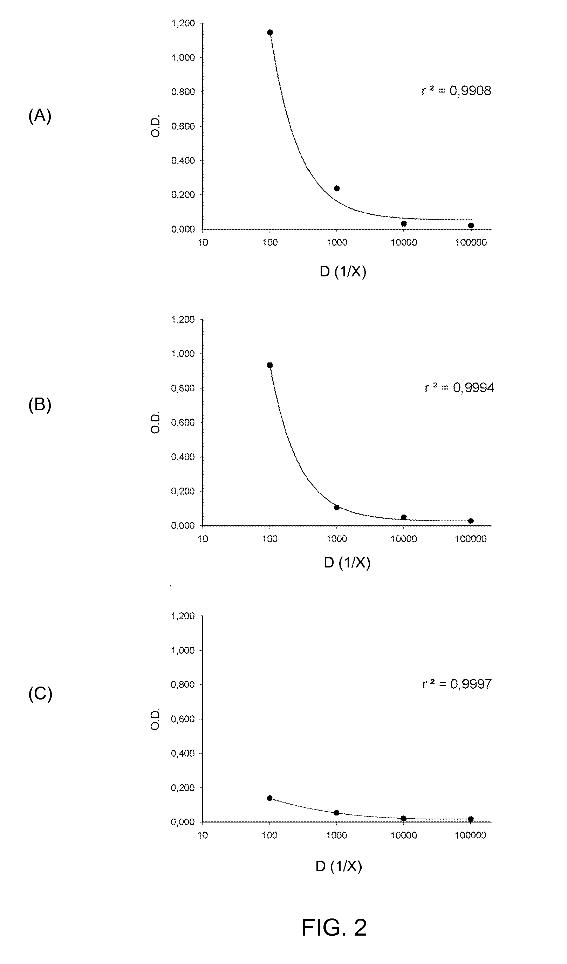

FIG. 2 corresponds to graphics with the titration of purified sera from rabbits immunized with peptide sequences SEQ ID NO 3 (A) or SEQ ID NO 1 (B). X-axis indicates the inverse (1/X) of the serum dilution (D). OD means Optical Density. As control, in (C) appears the titration of serum obtained from rabbits only immunized with the adjuvant and the carrier.

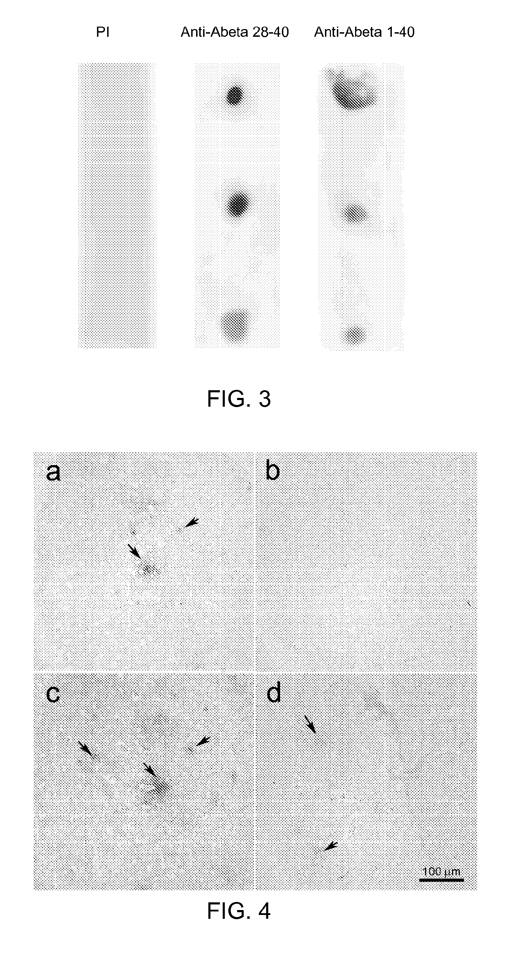

FIG. 3 is an image of a Dot blot assay. Dot blot detection of soluble Abeta with both the purified Anti-Abeta 28-40 peptide (anti-SEQ ID NO 3) and Anti-Abeta 1-40 peptide (anti-SEQ ID NO 1) antibodies. Rabbit pre-immune (PI) serum was used as control to determine the specificity of the raised antibodies.
FIG. 4 is an image with the immunohistochemical detection of Abeta plaques in the dog brain using both the purified Anti-Abeta 28-40 peptide and Anti-Abeta1-40 peptide antibodies. a) Immunostaining of Abeta plaques (arrows) detected with anti-Abeta 1-40 purified antibodies. b) Immunohistochemistry with pre-immune serum did not presented Abeta plaque immunodetection. c) Immunostaining of Abeta plaques (arrows) detected with anti-Abeta 28-40 purified antibodies. d) Immunostaining of Abeta plaques detected with commercial anti human Abeta peptide antibody, which presented low Immunoreactivity against canine plaques (arrows).

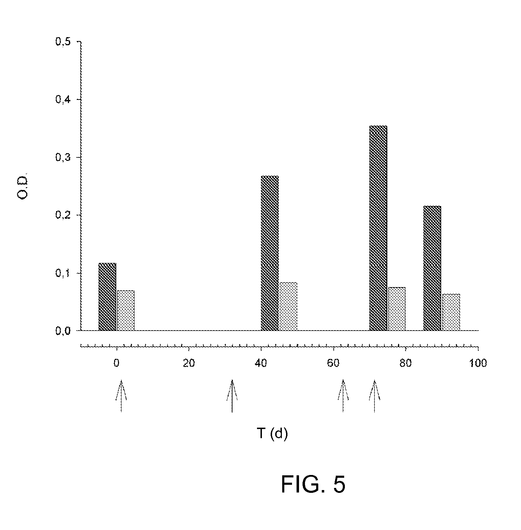

FIG. 5 is a graphic showing the anti-Abeta IgG titration of serum samples of dogs immunized (dark grey-bars) with Abeta 28-40 peptide (SEQ ID NO 3). Un-inmmunized dogs (light grey-bars) received injection of a mixture of adjuvant and carrier in the same proportion than immunized dogs. The arrows indicate the immunization days. In X-axis T (d) represents the blood extraction time in days. OD means Optical Density.

### DETAILED DESCRIPTION OF PARTICULAR EMBODIMENTS

The aim of the present invention is to provide peptide and antibodies derived from the administration of these peptides that can be used for preparing therapeutical agents for the treatment, prevention and/or diagnosis in a non-human mammal, of a disease assoiated with diffuse Abeta plaques depositions in the brain, said disease selected from the group consisting of Involutive Depression, Confusional Syndrome, Dysthymia and Cognitive Dysfunction Syndrome (CDS).

As it is shown in the examples below, the inventors surprisingly found that SEQ ID NO 1 or fragments derived from this peptide sequence elicit an immunological response in non-human mammals. Thus, these peptides can be used as agents for curing and/or preventing the above-mentioned diseases in non-human mammals. Moreover, said peptides have the additional advantage that they do not produce severe anaphylactic shocks or otherwise allergic reactions or other side effects in these animals.

Preferably, non-human mammals are selected from the group consisting of dogs, cats, monkeys, polar bears, rabbits, cows, sheep, pigs, guinea pigs, dolphins and Iberian lynxes, which have an Abeta peptide with a high homology degree to the Abeta 1-40 sequence of canine specie, which corresponds to SEQ ID NO 1.

The term "Abeta peptide" in this context includes the 39 and 40, amino acid peptides derived from the amyloid precursor protein (APP) of a non-human mammal, and any fragment of those peptides. All sequences mentioned in this document are fragments of canine Abeta peptide.

The invention provides the peptide SEQ ID NO 1 and fragments thereof as agent for curing, preventing and/or diagnosing in a non-human mammal a disease associated with diffuse Abeta plaques depositions in the brain, said disease selected from the group consisting of Involutive Depression, Confusional Syndrome, Dysthymia and Cognitive Dysfunction Syndrome (CDS).

Specifically, the invention provides the peptide which comprises SEQ ID NO 1 or fragments thereof as immunogenic agent for curing, preventing and/or diagnosing Cognitive Dysfunction Syndrome (CDS).

The peptides of this invention can be produced by any of the techniques know in the state of the art. Peptides can be chemically synthesized, or produced by conventional recombinant techniques.

In one embodiment of the invention the fragments derived from SEQ ID NO 1 comprise from 8 to 40 amino acids of said SEQ ID NO 1.

Preferably, the fragment is independently selected from the group consisting of SEQ ID NO 2, SEQ ID NO 3, SEQ ID NO 4, SEQ ID NO 5, SEQ ID NO 6, SEQ ID NO 7, SEQ ID NO 8 and SEQ ID NO 9.

In yet another embodiment the fragment is SEQ ID NO 2. This sequence corresponds to the C-terminal end of SEQ ID NO 1, which has been found to be less immunogenic than the N-terminal end of the Abeta 1-40 protein, as will be demonstrated below.

In yet a preferred embodiment, the fragment consists in SEQ ID NO 3. As will be shown in the examples below, the injection of SEQ ID NO 3 in dogs elicits a highly specific immunogenic response.

The peptide which comprises SEQ ID NO 1 and fragments thereof mentioned above are used to provide veterinary compositions comprising an immunological effective amount of one or more of these peptide sequences in a pharmaceutically acceptable delivery system.

The term "veterinary composition" is referred to a composition including the peptide corresponding to SEQ ID NO 1, and/or fragments consisting of at least 8 successive amino acids of SEQ ID NO 1, which are short linear peptides. The core sequence (8 amino acids) can be increased in size by addition of one or more amino acids to the N- terminal, the C-terminal or both sides, to form an immunogenic fragment derived from SEQ ID NO 1. Also, the veterinary composition can include a cocktail of two or more fragments which are immunogenic or not, and that can be used to induce immunogenicity. Further, a veterinary composition can include an immunogenic polypeptide chain composed by the peptide bond of two or more molecules of any fragment of SEQ ID NO 1, separated or not by one or more amino acids used as spacers, and by the peptide bond of one or more molecules of two or more fragments, being any of these units separated or not by one or more amino acids used as spacers.

The efficacy of the peptide sequence composition of the present invention can be established by injecting an animal, for example, a rabbit, with an immunogenic composition comprising the peptides sequences of the invention. The immune response to the sequences of the invention is monitored. A detailed description of the procedures is provided in the examples below.

Accordingly, the subject veterinary composition can be formulated as a vaccine using pharmaceutically acceptable adjuvants, carriers or other ingredients routinely employed in the formulation of vaccine compositions. Among the ingredients that can be used in this invention are adjuvants or emulsifiers including alum, liposyn, saponin, squalene, L121, emulsigen monophosphyryl lipid A (MPL), polysorbate 80, QS21, Montanide ISA51, ISA35, ISA206, and ISA720 as well as others available efficacious adjuvants and emulsifiers. The composition may be formulated for immediate release or sustained release. The composition may also be formulated for induction or systemic immunity, e.g., by entrapment in or co-administration with microplates. Such formulations are readily available to one of ordinary skill in the art.

The veterinary compositions of the present invention include as proper excipients or carriers those broadly known by the skilled man, including flavoring agents, sweeteners, buffers, preservatives, solubilizing agents, and stabilizing agents.

The veterinary compositions of the present invention can be administered via any conventional route such as, subcutaneous, oral, buccal, intraocular, intramuscular, parenteral, enteral, transdermal, depot, intravaginal or rectal administration, or in a form suitable for administration by inhalation or insufflation (either through the mouth, the nose or the ears). The vaccine compositions can be administered in a single dose or in multiple doses. A suitable immunization schedule is readily determined and available to one of ordinary skill in the art.

The veterinary composition of the present invention comprises an effective amount of one or more of the peptides of the present invention and a pharmaceutically acceptable carrier and /or adjuvant. Such a composition in a suitable dosage unit form generally contains about 0.0001 pg to about 250 g of the peptide sequences, as immunogenic agents, per animal. When delivered in multiple doses, the effective amount may be conveniently divided per dosage unit. For example, an initial dose, e.g. 0.0025-0.5 mg per animal; preferably 1-50 mg per animal of the immunogenic agent is to be administered by injection, preferably subcutaneous, followed by repeated doses of similar or different amounts. Dosage may vary with parameters like breed, age, weight, sex, and general health of the treated subject, as is well known in the vaccine and therapeutic arts.

The immune response to a veterinary vaccine composition can be improved by delivery through entrapment in or on biodegradable microparticles. The immunogenic agent can be encapsulated with or without an adjuvant, in or on biodegradable microparticles, to potentiate immune responses and to provide time-controlled release for sustained or periodic responses.

Said veterinary vaccine compositions are formulated in an appropriate way depending on the administration mode, including liquid formulations, such as injectable suspensions or solutions, syrups, capsules with the peptide sequences of the invention in dry form, dry powder that can be dissolved in water or any other liquid, suppositories, pills and tablets.

The veterinary compositions of the present invention, including either at least one peptide of the invention, can be complemented with an antioxidant therapy, commonly used for treating these kinds of diseases.

The administration of the peptides of the invention as immunogenic agents is an active immunization. The alternative to the active immunization with the peptide comprising SEQ ID NO 1 or fragments thereof as agents for the treatment, prevention and/or diagnosis in non-human mammals of a disease in which diffuse Abeta plaques exists, is the use of antibodies or fragments thereof raised by the peptides of the invention and that specifically recognize SEQ ID NO 1. This alternative method of immunization is called passive immunization, which involves administering performed antibodies from external sources to the recipient subject.

As exposed in the following examples, those antibodies that had been raised employing fragments derived from SEQ ID NO 1 or the SEQ ID NO 1 itself, and which can bind to Abeta peptides and/or β-amyloid plaques of aged canine with a cognitive impairment associated with the Cognitive Dysfunction Syndrome (CDS), are also useful as agents for treating, preventing and/or diagnosing said disease. Thus, the antibodies raised in this invention can be included in a pharmaceutical formulation that provides an effective method for the prevention, treatment and/or diagnosis of CDS in non-human mammals. Preferably the non-human mammals are selected from the group consisting of are selected from the group consisting of dogs, cats, monkeys, polar bears, rabbits, cows, sheep, pigs, guinea pigs, dolphins and Iberian lynxes. All these non-human mammals have an Abeta peptide sequence with a high homology to that of SEQ ID NO 1 (the canine Abeta 1-40 peptide sequence).

The antibodies of the invention can be raised by conventional techniques well known by the one skilled in the art, some of which are explained in the examples.

To improve the nature of the antibodies or fragments thereof, raised by SEQ ID NO 1 or any of its fragments, these can be modified by making them less immunogenic, for example by adapting them to the recipient specie. This is done with standard procedures, using as the constant part of the antibodies those parts of the recipient specie attached to the variable region of the antibodies raised by SEQ ID NO 1 or any of its fragments.

The veterinary compositions of this invention comprise effective therapeutic amounts of antibodies and pharmaceutically acceptable excipients suitable for administration to a subject presenting CDS, Involutive Depression, Confusional Syndrome or Dysthymia. Examples of acceptable excipients include buffers, surfactants, preservatives, solubilizing agents, isotonicity agents, stabilizing agents, and the like designed to be appropriate for the selected mode of administration. Such a composition in a suitable dosage unit form generally contains about 0.00001 pg to about 250 g of the antibody per animal. When delivered in multiple doses, the effective amount may be conveniently divided per dosage unit. For example, an initial dose, e.g. 0.0025-0.5 mg per animal of the antibody to be administered by injection, preferably subcutaneous, followed by repeated doses of similar or different amounts. Dosage may vary with breed, sex, age, weight, and general health of the canine subject as is well known in the vaccine and therapeutic arts.

A person skilled in the art would select an appropriate administration route for the antibodies of the present invention. Antibodies can be administered via any conventional route such as, subcutaneous, oral, buccal, intraocular, intramuscular, parenteral, enteral, transdermal, depot, intravaginal or rectal administration, or in a form suitable for administration by inhalation or insufflation (either through the mouth, the nose or the ears). The antibodies can be administered in a single dose or in multiple doses. A suitable treatment schedule is readily determined and available to one of ordinary skill in the art.

Animals that suffer from a disease which is characterized by diffuse Abeta plaques depositions in the brain will have soluble Abeta peptide fragments in Cerebrospinal fluid (CSF) or blood. Thus, the antibodies of this invention can be useful for a diagnostic method based on the detection of soluble Abeta peptides in CFS or blood by using said antibodies, which cross react with the Abeta petides as is shown in the following examples.

Yet another embodiment of this invention provides a kit that contains all necessary reagents to perform the diagnosis method mentioned above.

### EXAMPLES

### EXAMPLE 1

### SYNTHESIS OF PEPTIDE SEQUENCES OF THE PRESENT INVENTION

Two of the peptide sequences of the present invention, SEQ ID NO 1 and SEQ ID NO 3, corresponding to the canine Abeta protein from amino acids 1 to 40 (Abeta 1-40) and amino acids 28 to 40 (Abeta 28-40), respectively, were synthesized individually by the Merryfield solid-phase synthesis technique on Applied Biosystems automated peptide synthesizer (Model 433A Peptide Synthesizer) using Fluorenylmethyloxycarbonyl chloride (Fmoc) chemistry as protecting groups. After complete assembly of the desired peptide, the product was treated with trifluoroacetic acid to cleave the peptide from the resin where it was attached and deblock the protecting groups on the amino acid side chains. The cleaved, extracted and washed peptides were purified by HPLC using a Waters Delta Prep 4000 HPLC analyzer, and characterized by reverse phase HPLC with a Waters Delta 6000 HPLC apparatus. Finally the total amount of peptides was calculated by Mass Spectrometry by using an Applied Biosystems apparatus, model 4700 Proteomic analyzer.

In order to enhance its immunogeneicity, each peptide was additionally conjugated with Keyhole Limpet Hemocyanin (KLH), a standard carrier widely used in vaccine administration procedures. The resultant amount of KLH-conjugated peptide was split into aliquots of 0.5 mg peptide and 0.7 mg KLH each, lyophilized and stored at -20°C.

### EXAMPLE 2

### EVALUATION OF THE IMMUNOGENICITY OF THE PEPTIDE SEQUENCES OF THE PRESENT INVENTION

To design a veterinary vaccine composition that generates a high level of high affinity antibodies with cross reactivity to the soluble Abeta 1-40 peptide and the plaques in the CNS of dogs with CDS, the relative immunogeneicity of the synthesized sequences of example 1 were characterized separately. To do that, SEQ ID NO 1 and SEQ ID NO 3 were administered conjugated with KLH and mixed with 1 mg of Alum (Sigma, St Louis, U.S.A.) an adjuvant of general veterinary use. The relative immunegenicity of the two peptide sequences was compared according to standard titration of the immune products generated.

Experiments were performed in adult female New Zealand White rabbits weighing between 1.5-2 kg at the beginning of the study. They were kept on a 12h light/12 h dark cycle and housed with free access to food and water. All animals were manipulated according to the European legislation (86/609/EEC) for animal handling and experimentation. Procedures were approved by the Ethic Committee of the Universitat de Barcelona, under supervision of the Generalitat de Catalunya; all efforts were made to minimize animal suffering and to use only the number of animals necessary to produce reliable scientific data.

Rabbits were subcutaneously immunized with 100 µg of peptide from either:
a) SEQ ID NO 3 conjugated with KLH and 1 mg Alum used as adjuvant (n= 2);
b) SEQ ID NO 1 conjugated with KLH and 1 mg Alum used as adjuvant (n= 2);
or c) KLH and 1mg Alum (no peptide n= 2). The immunization protocol
consisted of a dose subcutaneously delivered up to six sites near the back of the neck, and repeated at days 11, 21, 31 and 41.

Samples of serum were collected from each rabbit at days 0, 21, 31 and 51. Each time, 5 ml blood samples were collected from the ear marginal vein in plastic tubes with no additive. At day 71 rabbits were anaesthetized and exanguinated. Blood samples were then kept at room temperature (RT) for 30 min., afterwards they were centrifuged for 10 min at 3000 rpm. Supernatants were collected as serum samples, aliquoted and stored at -80°C.

Titration of serum samples to detect the presence of antibodies that had been raised by SEQ ID NO 1 and SEQ ID NO 3 was performed by Enzime-Linked Immunosorbent Assays (ELISA) on 96-well plates developed by the biotin-avidin-peroxidase method. Plate sensitization was made overnight at 4°C with 2µg/ml of Abeta1-40 peptide diluted in 50 mM NaHCO₃, pH 9.6. After washing, plate blockade was performed with 0.05% PBS-T and 5% powdered milk for 2h at RT. Serum samples were applied at 4 different dilutions (1/100, 1/1000, 1/10000 i 1/100000) in PBS for 2 hours at RT. After washing, specific binding was detected by incubation with Biotin conjugated anti-Rabbit IgG antibody (Sigma, St. Louis, MO, U.S.A) diluted 1/20000 in TBS for 1 hour at RT. After washing and incubation with ExtrAvidin (Sigma, St. Louis, MO, U.S.A) samples were developed with tetramethylbenzidine for 10 min in the dark. Plates were densitometrically analized at 450 nm with a Tecan Sunrise Absorbance Reader apparatus (Tecan lberica, Madrid, Spain). Serum samples from day 0 (pre-immunized rabbit samples) were used as negative controls. Monoconal rabbit anti -Abeta 1-40/1-42 antibodies (Chemicon, Temecula, CA, USA) diluted 1/10000 in TBS were used as positive controls. Standarization of ELISA detections was performed by construction of a patron curve using Rabbit IgG at 4 dilutions (1/5, 1/25, 1/125 and 1/625). To assess the plate sticking, no sensitization of 12 wells was made in each plate. In all cases, serum antibody titration was performed by duplicated.

No antibodies were detected in any serum samples of control rabbits (treated with KLH and Alum) at any time-point. In these animals, the highest values of optic density were found at day 31 but they were similar to background.

As can be deduced from FIG. 1, from day 21 following the first immunization, a specific production of antibodies was detected in serum samples of rabbits injected with SEQ ID NO 3 (FIG. 1 A). The highest levels of immunization were detected in samples of day 31. Similar results were found in serum samples of rabbits immunized with the SEQ ID NO 1 (FIG. 1 B). Titration of antibodies from day 31 indicates that, for the SEQ ID NO 3, the serum dilution that presents half of the maximal binding (B_{1/2}) is the 1/611 one, whereas this value for SEQ ID NO 1 is detected at a dilution of1/240. This demonstrates that, in rabbits, the SEQ ID NO 3 presents a higher immunogenic response than the SEQ ID NO 1.

### EXAMPLE 3

### PURIFICATION OF HIGH AFFINITY ANTIBODIES FROM RABBIT SERUM

Together with the high level of high affinity antibodies that cross react with the canine Abeta 1-40 (SEQ ID NO 1) and Abeta 28-40 (SEQ ID NO 3), serum samples generally also present antibodies against immunogens and proteins not specifically related to an Abeta immune response. Thus, to assess the potential therapeutic efficacy in the clearance of soluble Abeta peptide and amyloid plaques in the dog CNS, it is firstly needed to specifically isolate the high affinity anti-Abeta 28-40 (or anti-SEQ ID NO 3) and anti-Abeta1-40 (or anti-SEQ ID NO 1) antibodies from these samples.

Antibody purification was made by affinity chromatography in columns of 4B Sepharose activated with BrCN (GE healthcare 15gr) according to standard procedures. Synthetic Abeta 1-40 peptide (SEQ ID NO 1) was linked to the BrCN and used as antigen for the affinity purification of antibodies. Anti-Abeta 28-40 antibodies were purified from rabbit serum samples presenting the highest level of titers, i.e. serum samples collected on day 31 after immunization (example 2). The same was true for serum samples used for purification of anti-Abeta 1-40 antibodies. Purified antibodies were firstly eluted with an acid solution of 100 mM glycine in PBS (pH 2.5) and, then with a basic solution of 100 mM glycine and 4 M urea in PBS (pH 9).

The purified antibodies were excipient by ELISA on 96-well plates developed by the biotin-avidin-peroxidase method. Plate sensitization was made overnight at 4°C with 2µg/ml of canine Abeta 1-40 peptide diluted in 50 mM NaHCO₃ pH 9.6. The ELISA procedure was then carried out as explained in example 2.

Titration of purified sera from rabbits immunized with canine Abeta 28-40 (SEQ ID NO 3) or with canine Abeta 1-40 (SEQ ID NO 1) peptides was compared with purification of serum obtained from rabbits only immunized with the adjuvant and the carrier (FIG 2 A-C). High titers of specific antibodies against canine Abeta1-40 were detected in serum from rabbits immunized with the Abeta 28-40 fragment (SEQ ID NO 3), as can be deduced from FIG. 2 (A), which corresponds to the titration of purified sera from rabbits immunized with this peptide sequence. Although slightly lower than the acid elution, high titers of anti-Abeta antibodies were also detected in the basic elution of the purification of Abeta 28-40 serum. Similar results were found in the purification of serum samples from rabbits immunized with the Abeta 1-40 peptide (SEQ ID NO 1), as derived from FIG. 2 (B), which is the titration of purified sera from rabbits immunized with peptide sequence SEQ ID NO 1.

Taken together, results from example 2 and example 3 clearly demonstrates that a standard pharmacological preparation of Abeta 28-40 peptide sequence (SEQ ID NO 3) or Abeta 1-40 peptide sequence (SEQ IN DO 1), formulated as a vaccine, induces in each case the production of a highly specific antibody against the canine Abeta 1-40 peptide.

### EXAMPLE 4

### EVALUATION OF PURIFIED ANTI-ABETA PEPTIDE ANTIBODIES BY CROSS-REACTIVITIY TO CANINE SOLUBLE ABETA.

To validate the two generated veterinary vaccine compositions of Example 3, which generate a high level of high affinity anti-Abeta antibodies with cross reactivity to the soluble Abeta peptide, it is necessary to evaluate the antigen cross-reactivity of the purified antibodies with the canine soluble Abeta. Thus, the purified anti-Abeta 28-40 and anti-Abeta 1-40 antibodies were separately used for immunodetection of canine soluble Abeta 1-40 peptide by dot blot experiments as follow.

Canine soluble Abeta 1-40 peptide at a concentration of 2 µg/cm² was directly incubated onto 0.2 µg pore polyvinylidene difluoride (PVDF) membranes (Amersham, UK). Membranes were washed in 10mM Tris-buffered saline with 0.1 % Tween 20 (TTBS). After the 1 h blocking step performed in the same buffer containing 5% milk/TTBS, and allowed to air dry for 20 min. Then 2 µl dots of purified anti-Abeta 1-40 peptide and anti-Abeta 28-40 peptide antibodies were separately blotted onto the activated membrane for 30 min. After washing with the previous buffer, an horseradish peroxidase-conjugated IgG goat anti-rabbit was applied at 1:10,000 (Bio-Rad, Hercules, CA) as secondary antibody and densitometricaly developed with the ECL Amersham kit.

As shown in FIG. 3 canine soluble Abeta 1-40 peptide was detected with both the purified Anti-Abeta 28-40 peptide (or anti- SEQ ID NO 3) and Anti-Abeta 1-40 peptide (or anti-SEQ ID NO 1) antibodies, whereas membrane dots incubated with the rabbit pre-immune serum showed no specific Abeta immunolabelling.

Thus, the antibodies raised in this invention can be included in a pharmaceutical formulation suitable for administration to a canine subject, which provides an effective method for immunoneutralization of soluble Abeta proteins in the CNS for the prevention and treatment of CDS in the dog and other non-human mammals that have Abeta proteins with high homology to that of dogs, such as cats, monkeys, polar bears, rabbits, cows, sheep, pigs, guinea pigs, dolphins and Iberian lynxes. As explained previously, the induction of plasma anti-Abeta or other antipeptide antibodies serves to quickly and efficiently increase the clearance of CNS Abeta. Generation of this peripheral sink mechanism is considered useful for treating abnormal Abeta protein accumulation in the CNS, because of the resulting net efflux of central Abeta to the periphery and its rapid elimination.

### EXAMPLE 5

### EVALUATION OF PURIFIED ANTIBODIES BY CROSS-REACTIVITIES TO DIFUSE AMYLOID PLAQUES OF AGED CANINE WITH CDS

Male and female dogs of various breeds and ages certified by their medical records from the veterinary hospital Ars Veterinaria of Barcelona, Spain, were used in this study. In all cases, donation was formally approved by the owner and euthanasia justified for medical reasons; the animals were sacrified with an I.V. overdose of sodium thiopental (75 mg/kg; Thiobarbital; Braun Medical S.A.). All animals were treated according to European legislation on animal handling and experiments (86/609/EU), and procedures were approved by the Ethical Committee of the University of Barcelona.

All efforts were made to minimize animal suffering and to use no more than the number of animals needed for reliable scientific data.

To evaluate the cognitive status of the dogs, a validated cognitive test was used in collaboration with pet owners. This test includes nine items: elimination behavior, life rhythm, walking, posture, playful and exploratory behaviors, interaction with other animals or with the owners, learning of specific behaviors and adaptive capabilities and was filled out by a veterinarian. For each item score 1 indicated the normality of the specific behavior, and scores 2, 3, 4 and 5 the degree of abnormal behavior. The final total score reflected the cognitive status of the animal. In some cases normality was qualified considering the diagnostic previously established by the veterinarian. Based on their score, dogs were categorized as young control, i.e. with no signs of cognitive disorder, light cognitive impairment and severe cognitive impairment. These signs are typical disorders for CDS.

Immediately after death, dog brains were quickly removed, and 1-cm-thick coronal sections of cerebral cortex were immersion fixed in 10% neutral buffered formalin for 4 weeks. After a 3-day cryoprotection, they were frozen on powdered dry ice, and 12- µm-thick serial sections of dorsal anterior prefrontal cortex corresponding to area 8a on the proreal gyrus were obtained with a cryostat. Once mounted on polyglycine-coated glass slides, they were kept at -40°C until use.

Series of sections of each brain were processed for immunohistochemical studies with the avidin-biotin complex (ABC) method employing the Vectastain Elite ABC kit (Vector Laboratories, Burlingame, CA). To unmask plaque immunoreactivity, sections were pretreated with 98-100% formic acid for 3 min at RT. Samples of purified anti Abeta antibodies from example 3 were used at dilutions of 1/100. For negative controls, sections were stained with omission of this antibody. After rinse, sections were incubated in 0.3% hydrogen peroxide in methanol for 10 min at RT to block endogenous peroxidases. Afterward, they were incubated in an immunobuffer solution of 3% normal goat serum (NGS) with 0.5% Triton X-1 00 in PBS for 1 hr at RT to block nonspecific reactions. Then, sections were incubated with the purified antibody overnight at 4°C Biotilynated goat anti-rabbit-IgG (1/200; Vector Laboratories) was used. Visualization of the antigen-antibody complex was performed with 1/100 dilution of avidin-biotin peroxidase with 3,3 α-diaminobenzidine peroxidase substrate kit (Vector Laboratories, U.S.A.). Adjacent brain sections were stained with the Bielschowsky's silver staining in order to reveal the presence of diffuse amyloid plaques and validate the specificity of the purified antibodies. Also, additional adjacent sections were counterstained with cresyl violet to reveal the canine brain cytoarchitecture.

Purified anti-Abeta antibodies raised against Abeta 28-40 and from Abeta 1-40 fragments specifically stained Abeta diffuse plaques in the dog brain sections, as can be seen in images c) and a), respectively, of FIG. 4. Brain parenchyma and neurons showed no specific staining. Control staining with the Bielschovsky's method revealed a very similar staining to the one observed by immunohistochemistry. The observed differences are explained by the known limitations of histological stainings (such as the Bielschowsky's one) of immature Abeta plaques of the canine brain (Shimada et al 1991). These results constitute clear pieces of evidence for the specificity of the raised and purified antibodies of the invention.

From FIG. 4 b), which corresponds to the immunohistochemistry with pre-immune serum of the dog brains, is deduced that the serum was not able to detect any Abeta plaque. Low immunoreactivity against canine plaques (arrows) was detected with commercial anti-human Abeta peptide antibody, as shown in FIG. 4 d).

Starting at the age of 8 years, and increasing with age and cognitive impairment severity, Abeta immunohistochemistry revealed the specific anti-Abeta 28-40 and anti-Abeta 1-40 antibodies binding to delicate and progressively more compact diffuse deposits. The deposits density and distribution throughout all cortical layers of the cerebral cortex coincide with the well-known four-stage distribution (I-IV) of the canine maturation process of diffuse amyloid plaques (Pugliese et al., "Gamma-aminobutyric acidergic interneuron vulnerability to aging in canine prefrontal cortex.", J. Neurosci. Res.-2004, 77:913-920). No plaques were detected in subcortical brain areas or the white matter.

An alternative therapy to active immunization with Abeta peptide for the treatment and/or prevention of cognitive deficits in non-human mammals is the passive administration of specific anti-Abeta antibodies. Passive immunotherapy allows customizing the antibody molecule to obtain one aimed at a specific epitope, and select the isotype of the antibody to be administered. Another advantage of the direct administration of a specific antibody is that its effectiveness does not depend on the immune system activation that is generally very low in aged animals. Another advantage of the no involvement of the immune systrem is that, in case of adverse reactions, passive immunotherapy immediate suspension or reduction of the treatment will directly stop these adverse effects.

Thus, the antibodies raised in this invention can be included in a pharmaceutical formulation suitable for administration to a canine subject, and provide an effective method for the clearance of amyloid plaques in the CNS for the prevention and treatment of CDS in the dog and other non-human mammals. As explained previously, the direct administration of anti-Abeta or other antipeptide antibodies serves to quickly and efficiently increase the clearance of CNS Abeta. Generation of this peripheral sink mechanism is considered useful for treating abnormal Abeta protein accumulation in the CNS, because of the resulting net efflux of central Abeta to the periphery and its rapid elimination.

Similarly, the antibodies can also be used for post-mortem diagnostics and in genetic studies to determine the similarities between the Abeta plaque composition and organization of different species.

### EXAMPLE 6

### THE IMMUNOGENICITY OF REPRESENTATIVE PEPTIDE SEQUENCES OF THE INVENTION IN DOGS AS IMMUNOTHERAPEUTIC AGENTS FOR CDS

To validate the immunogenicity, immunization of dogs with the synthesized Abeta 28-40 peptide (SEQ ID NO 3) was assayed, and antibody generation was assessed in serum samples by ELISA. To do that, the peptide fragment was administered conjugated with KLH and mixed with 1 mg Alum (Sigma, St. Louis, MO, U.S.A.), an adjuvant of general veterinary use. The immunogenicity of the Abeta 28-40 peptide was compared versus pre-immune serum according to standard titration of the immune products generated.

Male and female dogs of various breeds, sex and ages certified by their medical records from the veterinary hospital Ars Veterinaria of Barcelona, Spain, were used in this study. In all cases, pet owners formally informed approved the treatment of their dogs. All animals were treated according to European legislation on animal experimentation (86/609/EU), and procedures were approved by the Ethical Committee of the University of Barcelona. All efforts were made to minimize animal suffering and to use no more than the number of animals needed for reliable scientific data.

Dogs were subcutaneously immunized with either: a) 100 µg per animal of SEQ ID NO 3 conjugated with KLH and 1 mg Alum used as adjuvant (n= 2) or b) 100 µg per animal of KLH and 1 mg Alum (no peptide n= 2). The administered dose corresponds to a therapeutic amount from 0.00001 pg to 250 g of the antibody per animal. The immunization protocol consisted of a dose subcutaneously delivered up to four sites near the back of the neck, and repeated at days 31, 61, 75 (arrows in FIG. 5). Monitoring and supervising of the animals were performed weekly until day 100. Samples of serum were collected from each dog at days 0, 30, 60, 74 and 90. Each time, 5 ml blood samples were collected from the radial vein in plastic tubes with no additives. To isolate serum samples were kept at RT for 30 min, then were centrifuged for 10 min at 3000 rpm. Serum was collected, aliquoted and stored at -80°C. The titration of antibodies against SEQ ID NO 3 was performed by ELISA on 96-well plates developed by the biotin-avidin-peroxidase method as explained in Example 2. Plate sensitization was made overnight at 4°C with 2µg/ml of canine Abeta 1-40 peptide diluted in 50 mM NaHCO₃ pH 9.6.

No antibody was detected in any serum of control dogs (immunized with KLH and Alum) at any time-point (light-grey bars of FIG. 5). In these animals, the highest values of optic density were found at day 60 but they were not different from background.

At day 30 after the first immunization, a specific production of anti-Abeta antibodies was detected in serum samples of dogs injected with the Abeta 28-40 peptide (dark-grey bars in FIG. 5). The highest values of optic density were detected in samples of day 74.

These results clearly demonstrate that the Abeta 28-40 peptide injection in dogs induces a high specific immunogenic response to the canine Abeta peptide. Thus, the Abeta 28-40 peptide formulated in a standard veterinary vaccine composition suitable for administration to a canine subject provides an effective method to develop immunogenicity against Abeta peptide. These immunogens elicit the production of site-specific antibodies which bind to the soluble Abeta peptide, which immunoneutralizes the soluble Abeta-derived toxins and will facilitate the clearance of amyloid plaques in the dog CNS. No secondary effects were observed in the dogs, neither anaphylactic shocks, nor allergenic reactions, low of weight, etc.

Thus, the peptide sequences of this invention can be included in a veterinary vaccine formulation suitable for administration to a canine subject, with the aim of promoting an immune response with antibodies that can further immunoneutralize soluble Abeta proteins in the CNS for the prevention and treatment of CDS in the dog and other non-human mammals that have Abeta proteins with high homology to that of dogs, such as cats, monkeys, bears, rabbits, cows, sheep, pigs, guinea pigs, dolphins and Iberian lynxes.

### EXAMPLE 7

### APLICATION OF SOLUBLE ABETA DETECTION FOR CANINE COGNITIVE DYSFUNCTION SYNDROME DIAGNOSIS.

As performed in Example 4 with the purified anti-Abeta 28-40 and anti-Abeta 1-40 antibodies and the canine Abeta peptide immobilized in a PVP membrane, other assays with serum of dogs suspected of suffering CDS are made, using the anti-Abeta 28-40 (or anti-SEQ ID NO 3) and anti-Abeta 1-40 (or anti- SEQ ID NO 1) antibodies as diagnostic agents. All the dogs in which a positive dot blot is detected are good diagnosed and suffer CDS.

The antibodies of the invention are included in a kit that contains all necessary ingredients, reagents and/or elements to perform the diagnostic method by detecting increased soluble Abeta in cerebrospinal fluid (CSF), blood and other peripheral fluids or tissue samples.

As has been demonstrated by way of these examples, the peptide sequence SEQ ID NO 1 and fragments thereof, i.e. SEQ ID NO 3, induce high immune responses without prejudicial secondary effects when administered in dogs suffering from CDS. Thus, the present invention supposes an improvement of the current therapies used in the veterinarian field for curing those diseases, in which a cognition dysfunction exists due to the deposition of diffuse Abeta-plaques in the CNS, specifically in the brain, of the animals.

## Claims

1. A peptide which comprises SEQ ID NO 1 or fragments thereof as agent for curing, preventing and/or diagnosing in a non-human mammal a disease associated with diffuse Abeta plaques depositions in the brain, said disease selected from the group consisting of Involutive Depression, Confusional Syndrome, Dysthymia and Cognitive Dysfunction Syndrome (CDS).

2. The peptide of claim 1 which is SEQ ID NO 1 or fragments thereof as agent for curing, preventing and/or diagnosing in a non-human mammal a disease associated with diffuse Abeta plaques depositions in the brain, said disease selected from the group consisting of Involutive Depression, Confusional Syndrome, Dysthymia and Cognitive Dysfunction Syndrome (CDS).

3. The peptide according to any of claims 1-2, wherein the non-human mammal is selected from the group consisting of dogs, cats, monkeys, bears, rabbits, cows, sheep, pigs, guinea pigs, dolphins and Iberian lynxes.

4. The peptide according to any of claims 1-3 as agent for curing, preventing and/or diagnosing Cognitive Dysfunction Syndrome (CDS).

5. The peptide according to any of claims 1-4, wherein the fragment comprises from 8 to 40 amino acids of SEQ ID NO 1.

6. The peptide of claim 5, wherein the fragment is independently selected from the group consisting of SEQ ID NO 2, SEQ ID NO 3, SEQ ID NO 4, SEQ ID NO 5, SEQ ID NO 6, SEQ ID NO 7, SEQ ID NO 8 and SEQ ID NO 9.

7. The peptide according to claim 6, which is SEQ ID NO 2.

8. The peptide according to claim 6 which is SEQ ID NO 3.

9. A veterinary composition comprising effective therapeutic amounts of any of the peptide sequences as defined in any of claims 1-8, or mixtures thereof, and any acceptable carrier, excipient and/or adjuvant.

10. Antibody or fragments thereof raised by any of the peptide sequences described in any of claims 1-8 which specifically interact with SEQ ID NO 1 as agents for curing, preventing and/or diagnosing in a non-human mammal a disease associated with diffuse Abeta plaques depositions in the brain, said disease selected from the group consisting of Involutive Depression, Confusional Syndrome, Dysthymia and Cognitive Dysfunction Syndrome (CDS).

11. The antibodies or fragments thereof according to claim 10, wherein the non-human mammal is selected from the group consisting of dogs, cats, monkeys, bears, rabbits, cows, sheep, pigs, guinea pigs, dolphins and Iberian lynxes.

12. The antibodies or fragments thereof according to any of claims 10-11 as agents for curing, preventing and/or diagnosing Cognitive Dysfunction Syndrome (CDS).

13. A method of obtaining an antibody that specifically interacts with SEQ ID NO 1, which comprises the steps of:
a) administering in a non-human mammal a peptide which comprises SEQ ID NO 1 or fragments thereof; and
b) obtaining the antibody from the serum of said non-human mammal.

14. A veterinary composition comprising effective therapeutic amounts of antibodies of any of the claims 10-12, or mixtures thereof and any acceptable carrier, excipients and/or adjuvant.

15. Use of the antibodies according to claims 10-11 for the preparation of an agent for the diagnosis or prognosis of a disease associated with diffuse Abeta plaques depositions in the brain, said disease selected from the group consisting of Involutive Depression, Confusional Syndrome, Dysthymia and Cognitive Dysfunction Syndrome (CDS) in non-human mammals.


SEQUENCE LISTING

<110>  MEDIVET PHARMA, S.L.

<120>  Veterinary immunotherapy compositions for the Aged Related
       Cognitive Dysfunction

<130>  P1146EP00

<160>  9

<170>  PatentIn version 3.5

<210>  1
<211>  40
<212>  PRT
<213>  Canis lupus

<400>  1

Asp Ala Glu Phe Arg His Asp Ser Gly Tyr Glu Val His His Gln Lys
1               5                   10                  15

Leu Val Phe Phe Ala Glu Asp Val Gly Ser Asn Lys Gly Ala Ile Ile
            20                  25                  30

Gly Leu Met Val Gly Gly Val Val
        35                  40

<210>  2
<211>  8
<212>  PRT
<213>  Canis lupus

<400>  2

Gly Leu Met Val Gly Gly Val Val
1               5

<210>  3
<211>  13
<212>  PRT
<213>  Canis lupus

<400>  3

Lys Gly Ala Ile Ile Gly Leu Met Val Gly Gly Val Val
1               5                   10

<210>  4
<211>  9
<212>  PRT
<213>  Canis lupus

<400>  4

Ile Gly Leu Met Val Gly Gly Val Val
1               5

<210>  5
<211>  10
<212>  PRT
<213>  Canis lupus

<400>  5

Ile Ile Gly Leu Met Val Gly Gly Val Val
1               5                   10

<210>  6
<211>  11
<212>  PRT
<213>  Canis lupus

<400>  6

Ala Ile Ile Gly Leu Met Val Gly Gly Val Val
1               5                   10

<210>  7
<211>  12
<212>  PRT
<213>  Canis lupus

<400>  7

Gly Ala Ile Ile Gly Leu Met Val Gly Gly Val Val
1               5                   10

<210>  8
<211>  14
<212>  PRT
<213>  Canis lupus

<400>  8

Asn Lys Gly Ala Ile Ile Gly Leu Met Val Gly Gly Val Val
1               5                   10

<210>  9
<211>  15
<212>  PRT
<213>  Canis lupus

<400>  9

Ser Asn Lys Gly Ala Ile Ile Gly Leu Met Val Gly Gly Val Val
1               5                   10                  15